# EUROPEAN PATENT SPECIFICATION

(11) **EP 1 013 766 B1**
(45) Date of publication and mention of the grant of the patent: **03.11.2004**
(21) Application number: 99309491.1
(22) Date of filing: 29.11.1999
(51) Int. Cl.: C12N 15/49, C07K 14/16, C07K 16/10, C12N 5/10, G01N 33/569, G01N 33/68

(54) **Peptides for the detection of HIV-1-Group O**
Peptide zum Nachweis von HIV-1-Guppe O
Peptides pour la detection du HIV-1 de groupe O

(30) Priority: 30.11.1998 US 110292; 08.02.1999 US 119138; 04.11.1999 US 433428
(43) Date of publication of application: 28.06.2000
(73) Proprietor: Ortho-Clinical Diagnostics, Inc., Rochester, NY 14626-5101 (US)
(72) Inventor: De Leys, Robert, Three Bridges, NJ 08887-2128 (US); Zheng, Jian, Raritan, NJ 08869 (US)
(74) Representative: Mercer, Christopher Paul

(56) References cited:
- EP-A- 0 591 914
- WO-A-86/06414
- WO-A-96/12809
- WO-A-96/27013
- WO-A-98/45323
- J. EBERLE ET AL.: "Diversity of the immunodominant epitope of gp41 of HIV-1 subtype O and its validity for antibody detection" JOURNAL OF VIROLOGICAL METHODS, vol. 67, 1997, pages 85-91, XP002204876
- J. VAN BINSBERGEN ET AL.: "Reactivity of a new HIV-1 group O third generation A-HIV-1/-2 assay with an unusual HIV-1 seroconversion panel and HIV-1 group O/group M subtyped samples" JOURNAL OF VIROLOGICAL METHODS, vol. 69, 1997, pages 29-37, XP002204877

## Description

### Background

The principal etiological agent responsible for causing what has come to be known as acquired immunodeficiency syndrome (AIDS) is a non-transforming retrovirus belonging to the lentivirus genus (1). This virus, referred to as Human Immunodeficiency Virus type I (HIV-1), is now widely disseminated and constitutes a serious threat to health and productivity worldwide. Virtually all industrialized countries, as well as many in the developing world, now mandate the testing of blood donations to prevent the further transmission of this virus and the spread of disease through the use of contaminated blood and blood products. A related, genetically distinct, but less wide-spread and pathologically less aggressive virus capable of inducing similar disease was reported in 1986, and is referred to as HIV-2 (2). While HIV-2 is found primarily in West Africa and is less widely disseminated than HIV-1, many countries require the screening of blood donations for antibodies to this virus as well. An HIV-1 is disclosed in EP-178 978, while an HIV-2 is disclosed in EP-0 239 425.

One feature which is characteristic of human immunodeficiency viruses is their sequence variability. The genomes of all HIVs encode the enzyme reverse transcriptase. This enzyme, which the virus requires to convert its RNA genome into its double-stranded DNA equivalent prior to integration into host cell DNA, is essential for virus replication. Unlike many polymerases, this Mg⁺²-dependent enzyme lacks a 3'→5' exonuclease activity which normally serves a proofreading function. As a consequence, this enzyme tends to be error-prone. Within any HIV-infected individual, many naturally occurring sequence variants of the virus can be found, not all of which are viable. This observation has given rise to the notion of the quasi-species, a term used to describe a particular strain of HIV infecting an individual as a collection of all its closely related, naturally occurring sequence variants (3).

In addition to naturally occurring sequence variants within an infected individual, phylogenetic analyses of HIV-1 strains collected from all over the world have demonstrated that these strains can be grouped into at least 9 types (A - I) based on the similarity of their sequences (4). The differences between the types are greater than the differences observed between individual virus variants within a single infected person, or the differences between other variants belonging to the same type. The geographical distribution of these HIV-1 types varies significantly, with certain types being prevalent in one particular geographic region but rare or absent in another. Collectively, these HIV-1 types may be considered to form a group, which is usually referred to as group M (major).

In 1987, a highly divergent variant of HIV-1 was isolated that was immunologically easily distinguishable from commonly encountered HIV strains (5). This variant is described in EP-0 345 375, US Patents 5,304,466, and US 5,567,603. This virus (ANT70) was antigenically closer to HIV-1 reference strains than it was to HIV-2, but was nevertheless clearly very different. The sequence of the entire provirus was subsequently determined (6). While the genome organization of this virus confirmed that this isolate was an HIV-1, a comparison of its sequence with those of many other reference strains showed that this virus was highly divergent, and phylogenetic analyses placed this isolate in its own unique branch of the HIV phylogenetic tree.

In 1991, a second, highly divergent HIV-1 strain (MVP5180) was isolated and described (7). This isolate was disclosed in EP-0 591 914, and was found to cluster phylogenetically with ANT70. The genetic distance between these two isolates was approximately as great as the distance between the virus types belonging to group M. Together, these two isolates defined a new group of HIV-1 isolates. Because these isolates clustered outside the normal cluster of conventional HIV-1 isolates, they represented a new group, usually referred to as group O (outlier).

In 1992, a third person was identified in France who was infected with a group O strain (8). The sequence of the immunologically important viral *env* gene was determined, and is described in WO 96/12809. Subsequently, several additional group O-infected patients were identified in France (9), and the sequence of portions of the viral *env* proteins of these isolates was also determined. These sequences have been described in PCT/FR96/00294. An analysis of all of the available sequences showed that they cluster together in the branch of the HIV-1 phylogenetic tree corresponding to group O. Unlike group M, there seems to be little evidence for the existence of discrete virus types within the outlier group. With the exception of the French VAU isolate, virtually all of the group O isolates to date share a link to West-Central Africa. In this portion of Africa, it has been estimated that between 5% and 8% of all cases of HIV-1 infection are caused by group O variants, however, these percentages are strongly dependent on the specific geographical region (10, 11).

While there seems to be no significant differences between group M and group O strains in terms of pathology or disease progression, the detection of antibodies produced in response to a group O infection can be unreliable when the antigens used for serological testing are derived exclusively from group M strains (12, 13). Although antibodies produced to group O antigens will often cross-react with the corresponding group M antigens, the sensitivity for anti-group O antibodies can be significantly improved by incorporating a group O antigen into the test.

Although the existence of HIV-1 groups and types is well-established, an increasing number of isolates have been identified that cannot be conveniently assigned to a specific HIV-1 group M type. Through sequence analysis it has been possible to demonstrate that these isolates are the products of recombination between viruses belonging to two or more different types. In some cases, multiple recombination events must have occurred, giving rise to "mosaic" genomes. Multiple types have been shown to coexist within a single patient, and there have been reports of multiple group M types coexisting in a patient together with a group O strain (14, 15). Since the genomes of group M and group O strains also share regions which are very highly conserved, legitimate recombination could presumably occur between these viruses as well.

A preferred antigen for the detection of antibodies produced in response to HIV infection is the transmembrane portion of the viral envelope protein. This protein, referred to as gp41, is cleaved from a gp160 precursor in the infected cell by a cellular protease. This protein contains the viral fusion peptide at its N-terminus, which the virus needs in order to fuse with and penetrate a new host cell. It also provides an anchor for the surface envelope glycoprotein gp120, which is responsible for recognizing CD4 molecules and co-receptors for the virus on the surface of susceptible cells. The interaction between gp120 and gp41 is, however, non-covalent and somewhat labile. The gp41 protein is itself anchored in the viral or host cell membrane via a hydrophobic membrane-spanning region.

Little is known of the detailed three-dimensional structure of this protein. A limited amount of structural information concerning the extracellular domain of this protein is available from the Brookhaven Protein Data Base. However, the immunologically most relevant portion of gp41 is absent, probably because it is too mobile to give rise to reflections. A comparison of viral gp41 amino acid sequences corresponding to this immunologically important region reveals the presence of several extremely highly conserved amino acids in what is presumably the top of a tight disulfide-stabilized loop, suggesting that these amino acids serve an essential structural and functional role.

### Summary of the Invention

This invention relates to peptides, their preparation and use. Each peptide has a sequence that corresponds to the immunodominant region of the HIV-1 group O gp41 envelope protein. The sequence is characterized in that it does not correspond to any known naturally occurring group O variant.

In another aspect, this invention relates to a peptide, as described above, that is antigenic. Preferentially it binds anti-HIV-1 group O antibodies.

In yet another aspect, this invention relates to the use of the peptide, as described above, for the detection of antibodies produced in response to HIV 1 group O infection.

In yet another aspect, this invention relates to compositions and kits for determining the presence of HIV-1 group O antibodies, comprising the peptide as described above or its analog.

In yet another aspect, this invention relates to anti-HIV-1 group O antibodies and their production using the peptides, and their use in detecting HIV-1 antigens and HIV-1 infection.

A peptide referred to herein as "peptide 147" comprising the following amino acid sequence: NQQRLNSWGCKGRIICYTSARWH, wherein the amino-terminus is at the left of the sequence. The peptide may be modified chemically (16) at either end to endow it with properties that will facilitate its use, such as N-terminal acetylation, biotinylation, or the addition of a spacer arm to provide physical distance between the peptide and a functional group used to anchor the peptide to a solid phase or a carrier.

In addition to peptide 147 several variations of the amino acid sequence of peptide 147 were found to be useful.

A preferred peptide related 147 comprises the following amino acid sequence: XQQRLNSWGCKGRIICYTSARWH, wherein X can be any natural amino acid other than L-asparagine or a non-natural amino acid, that is, one that is not among the 20 recognized naturally occurring amino acids; but which may occur naturally or be of human design.

Another peptide related to 147 comprises the following amino acid sequence: ETLMQXQQRLNSWGCKGRIICYTSARWH, wherein X denotes any natural amino acid other than L-asparagine, or a non-natural amino acid.

Each of the preferred peptides may be modified chemically at their the amino or carboxyl terminus to endow it with properties that will facilitate its use such as, but not limited to, N-terminal acetylation, biotinylation, or the addition of a spacer group to provide physical distance between the peptide and a functional group used to anchor the peptide to a solid phase or a carrier.

The invention further relates to the preparation of a mosaic. A mosaic is a recombinant group M gp41 protein in which the group M immunodominant region has been replaced by an O-like immunodominant sequence. An α-helical antigenic region located downstream from the immunodominant region was also modified in a way to increase its likelihood of being recognized by anti-group O antibodies, while still retaining those amino acids required for inter-helix interactions and structural stabilization of the protein. The resulting recombinant is therefore an artificially constructed group M/group O hybrid that does not exist in nature.

In another aspect, this invention relates to the use of a recombinant protein, as described above, for the detection of antibodies produced in response to HIV-1 group O infection.

In another aspect, this invention relates to a recombinant protein, as described above, that preferentially binds anti-HIV-1 group O antibodies.

In yet another aspect, this invention relates to compositions and kits for determining the presence of HIV-1 group O antibodies, comprising a recombinant protein as described above.

In yet another aspect, this invention relates to anti-HIV-1 group O antibodies and their production using the recombinant protein, and their use in detecting HIV-1 antigens and HIV-1 infection.

A group O replacement is: RARLQALETLMQNQQRLNSWGCKGRIICYTSARWH, wherein the amino-terminus is at the left of the sequence.

The recombinant protein may be modified chemically at either end to endow it with properties that will facilitate its use, such as N-terminal acetylation, biotinylation, or the addition of a spacer arm to provide physical distance between the protein and a functional group used to anchor it to a solid phase or a carrier.

In yet another aspect, this invention relates to compositions and kits comprising a peptide or recombinant protein, as described above, and one or more antigens directed to other anti-HIV-1 type antibodies.

In yet another aspect, this invention relates to methods for detecting HIV-1 infection using a peptide or recombinant protein, as described above, in combination with one or more antigens directed to other HIV-1 type antibodies.

### Brief Description of the Drawings

- Figure 1.: Alignment of HIV-1 group O immunodominant region amino acid sequences.
- Figure 2.: Recognition of peptides 147 and 80 by an HIV-1 group O sample.
- Figure 3.: Ectodomain of gp41 (MN isolate)
- Figure 4.: Alignment of MN (type B) and group O gp41 sequences in the region of the descending helix
- Figures 5 a-c.: The stepwise reconstruction and replacement of the immunodominant region using overlapping synthetic oligonucleotides.

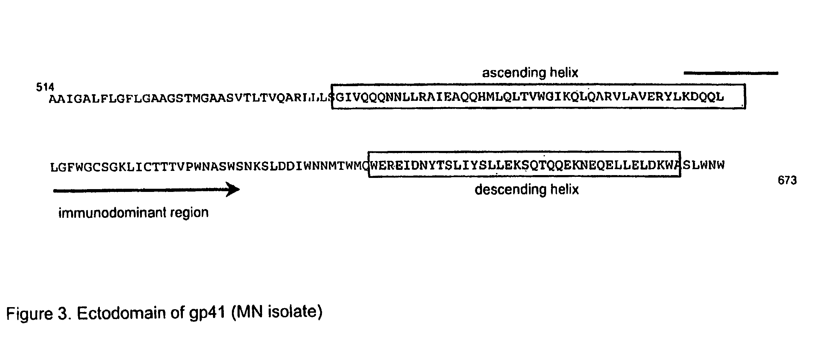

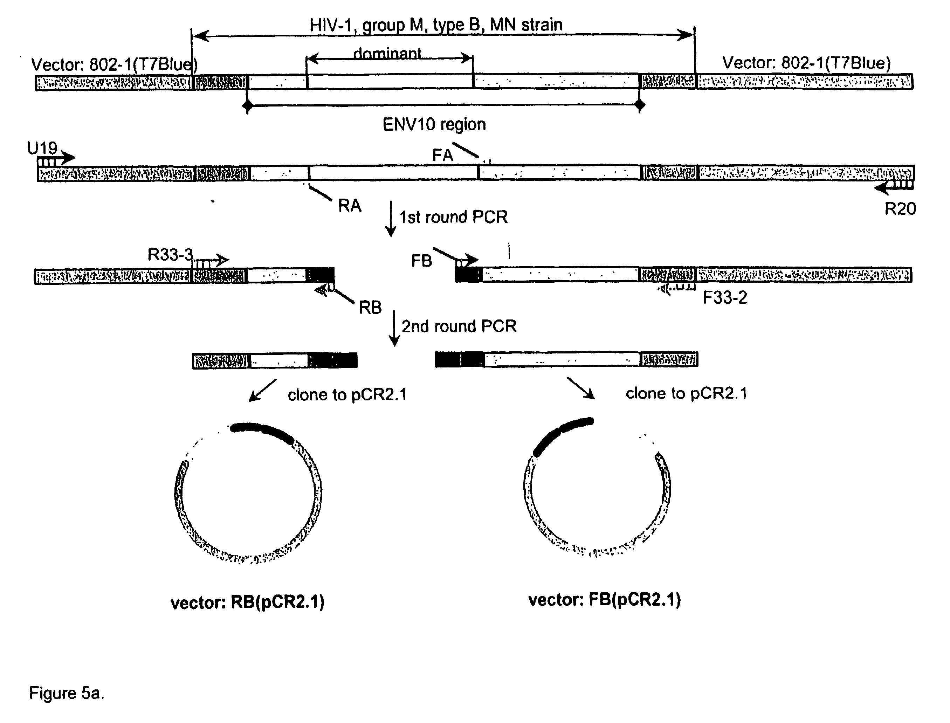

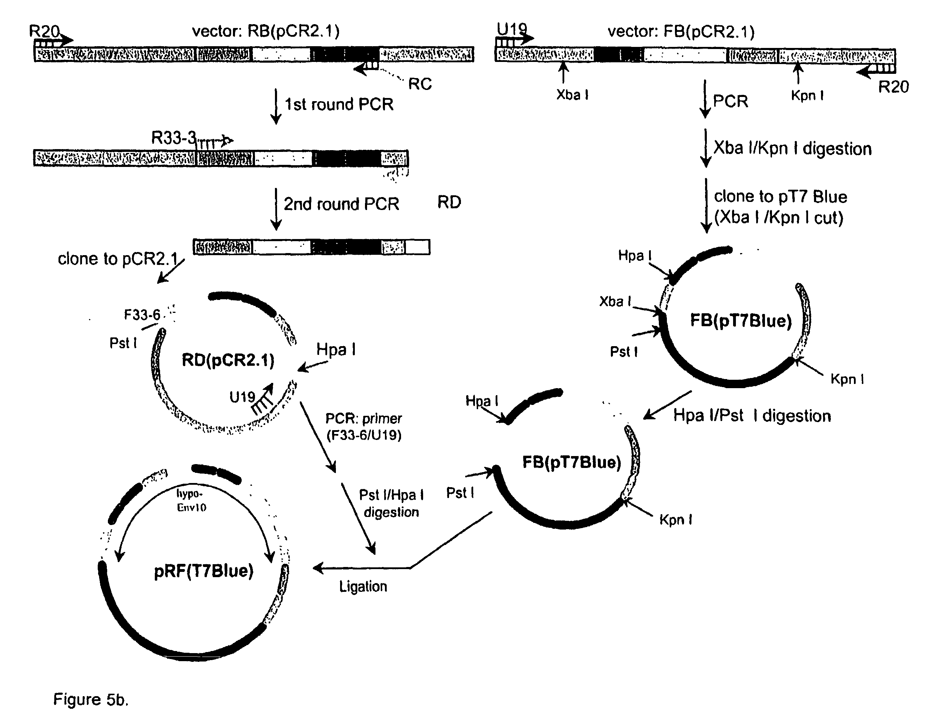

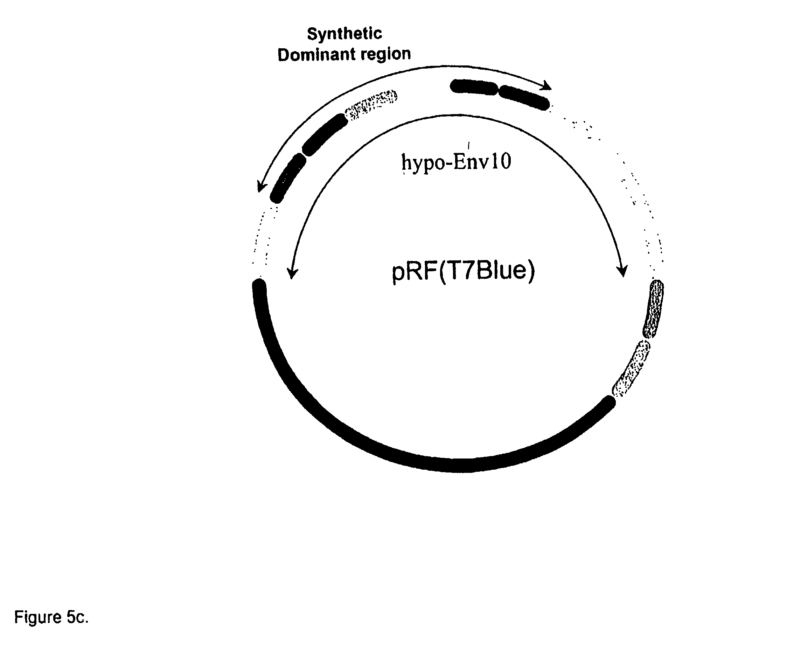

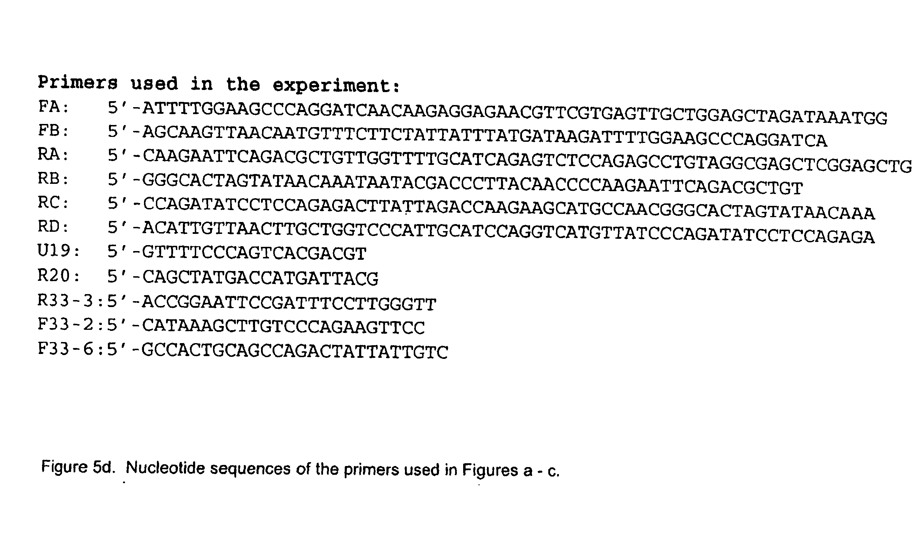

- Figure 5 d.: The nucleotide sequence of the primers used in Figures 3 a-c.

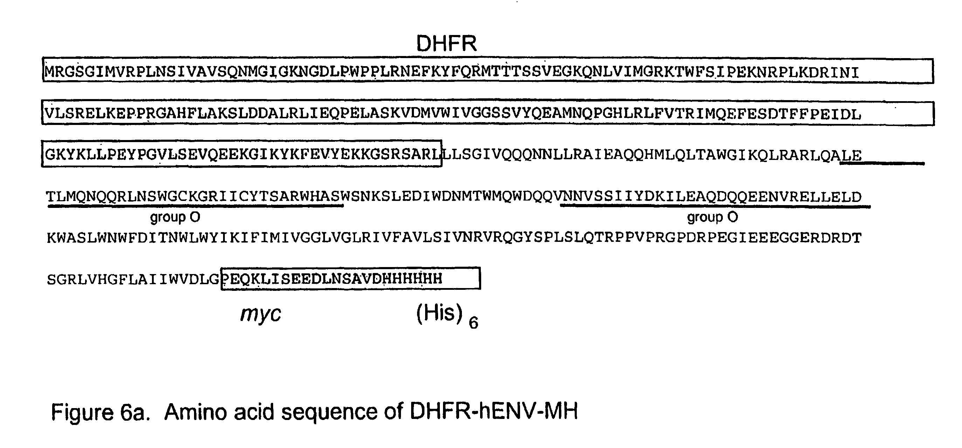

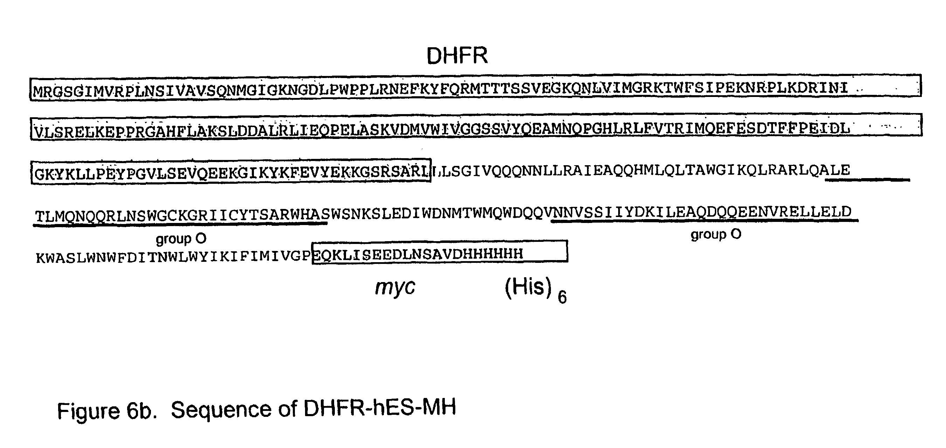

- Figure 6 a: The amino acid sequence of DHFR-hENV-MH.
- Figure 6 b.: The amino acid sequence of DHFR-hES-MH.

### Detailed Description of the Invention

A "sample" as used herein, refers to any substance which may contain the analyte of interest. A sample can be biological fluid, such as whole blood or whole blood components including red blood cells, white blood cells, platelets, serum and plasma, ascites, urine, cerebrospinal fluid, and other constituents of the body which may contain the analyte of interest

An "antibody" as used herein may be may be monoclonal or polyclonal and may be of any species of origin, including for example, human, rat, mouse, rabbit, horse, sheep, or may be chimeric antibodies. The antibodies may be recombinant monoclonal antibodies or chemically constructed. See M. Walker et al. Molec. Immunol. 26, 403-11 (1989); U.S. Patent 4,474,893; U.S. Patent 4,816,567; and U.S. Patent 4,676,980.

An "antigen" as used herein refers to any substance that as a result of coming in contact with appropriate tissues of an animal body, induces a state of sensitivity and/or resistance to infection. "Antigenic" means immunogenic or having the properties of an antigen, including but not limited to the generation of antibodies that bind the antigen. Antigens can be proteins, oligopeptides, or polypeptides and can be prepared using recombinant technology as well as synthetic methods.

For the purposes of the present invention it should be noted that because the genetic code is degenerate, more than one codon may be used to encode a particular amino acid, and therefore, the amino acid sequence can be encoded by any of a set of similar DNA oligonucleotides.

Furthermore, it is known that there is a substantial amount of redundancy in the various codons which code for specific amino acids. Therefore, this invention is also directed to those DNA sequences which contain alternative codons which code for the eventual translation of the identical amino acid. For purposes of this specification, a sequence bearing one or more replaced codons will be defined as a degenerate variation. Also included within the scope of this invention are mutations either in the DNA sequence or the translated protein which do not substantially alter the ultimate physical properties of the expressed protein. For example, substitution of valine for leucine, arginine for lysine, or asparagine for glutamine may not cause a change in functionality of the peptide.

Following expression of the peptide in a recombinant host cell several peptide purification procedures are available and suitable for use. For example, the peptide may be purified from cell lysates and extracts, or from conditioned culture medium, by various combinations of, or individual application of salt fractionation, ion exchange chromatography, size exclusion chromatography, hydroxylapatite adsorption chromatography and hydrophobic interaction chromatography . In addition, recombinant peptides can be separated from other cellular proteins by use of an immunoaffinity column made with monoclonal or polyclonal antibodies specific for full length peptides, or fragments.

Examples are set forth below. The example are intended to illustrate and not limit the invention.

### Example 1

**Sequences.** To determine which amino acids were characteristic of group O strains, gp41 sequences from as many different isolates possible (complete and partial) were collected and aligned. Many of these sequences were available from the scientific literature, while others were determined in this laboratory. Attention was focused towards the region of gp41 known to be immunodominant and of particular diagnostic value. The sequences used for the comparison were obtained from the following sources:

**Table 1.**

| Sources of HIV-1 group 0 sequences | |
|---|---|
| Sequence | reference |
| ANT70 | 6 |
| MVP5180 | 7 |
| VAU | 8 |
| DUR | WO 96/12809 |
| POC, FAN, LOB, MAN, NAN, ESS, NKO. BCF09. BCF12, BCF13. BCF14 | PCT/FR96/00294 |
| 686 | 17 |
| ABT063, ABT124, ABT1123, ABT2156, 193Ha | 18 |
| CDC7755, CDC1897 | 19 |
| HLD28.1515.1516. D47-2d, HCYT2c, Nr42. PE41 | this laboratory |

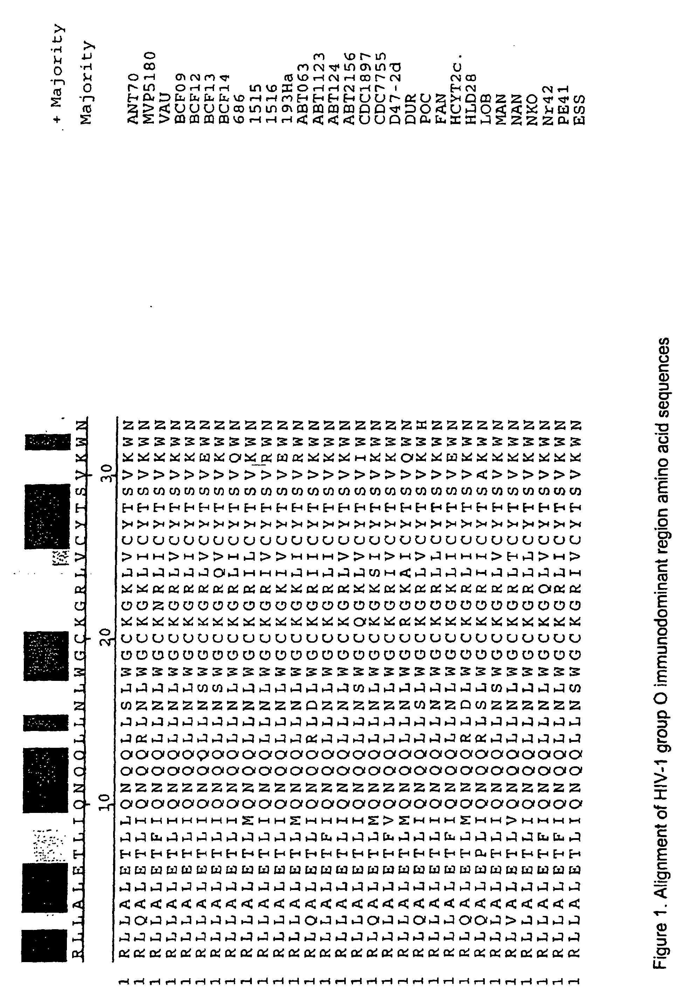

Viral amino acid sequences were trimmed to a 33 amino acid-fragment containing the immunodominant region of gp41 and were aligned using the program MEGALIGN (DNAStar). The alignment is shown in Figure 1.

The height of the column above each amino acid position shows graphically how well the amino acid in each position is conserved. Of the 33 positions in the region of interest, 18 amino acids are perfectly conserved in all strains. In the other 15 positions that are less well conserved, a closer examination of the amino acids found in these positions reveals that there is frequently a preference for amino acids with certain characteristics. For example, in positions 9 and 25, there is a strong preference for an amino acid with a hydrophobic side chain. There is also a strong preference for a lysine residue in position 21, and a positively charged amino acid in position 23.

Because it is known that the immunodominant region is primarily situated between amino acids 11 and 33 in the region shown in Table 3, a peptide sequence was derived in which an amino acid is incorporated in each position that can also be found in a naturally occurring strain. However, the resulting sequence is unique and does not correspond to that of any known group O virus. This sequence is approximately 62% identical to that of the original ANT70 isolate.

The amino acid sequence of the peptide (peptide 147) is as follows:

NQQRLNSWGCKGRIICYTSARWH

The following sequence alignment shows the similarities and differences between this sequence and the sequence of the original ANT70 isolate:

ANT70 NQQLLSLWGCKGKLVCYTSVKWN
peptide 147 NQQRLNSWGCKGRIICYTSARWH

**Peptide synthesis.** Peptide synthesis was carried out using 9-fluorenylmethoxycarbonyl (Fmoc) chemistry on commercially available polystyrene:divinylbenzene-crosslinked resin bearing the modified Rink linker in order to generate peptides with a C-terminal amide following cleavage. Protected amino acids were activated *in situ* using an equimolar quantity of N-[(dimethylamino)-1*H*-1,2,3-triazolo[4,5-b]pyridin-1-ylmethylene]-N-methylmet hanaminium hexafluorophosphate N-oxide (HATU) and 1-hydroxy-7-azabenzotriazole (HOAt), and a 2-fold molar excess of N-methylmorpholine (NMM) in N-methylpyrrolidone (NMP). A 4-fold excess of activated amino acid was used with respect to the loading capacity of the resin. Trityl (Trt) side-chain protection was used for glutamine, asparagine, cysteine, and histidine. The hydroxyl functions of serine, threonine, and tyrosine were protected with the *t*-butyl(*t*Bu)group, the side chain of lysine was protected with the *t*-butyloxycarbonyl (*t*Boc) group, and 2,2,5,7,8-pentamethylchroman-6-sulfonyl (Pmc) group was used for the side chain of arginine. All other amino acids were coupled without side chain protection.

Removal of the Fmoc group at the beginning of each cycle was achieved by treating the resin-bound peptide with a mixture of 2% 1,8-diazabicyclo[5.4.0]undec-7-ene and 2% piperidine in NMP for 15 minutes.

To facilitate the binding of the peptide to the solid phase for testing, the peptide was synthesized with a biotin residue at its amino terminus. Two glycine residues were added to the N-terminus of the peptide to serve as a spacer arm before attaching the biotin. Biotin was coupled by *in situ* activation of its carboxyl group using an equimolar quantity of O-(benzotriazol-1-yl)-1,1,3,3-tetramethyluronium tetrafluoroborate (TBTU) and a 2-fold molar excess of NMM. The biotin was dissolved in 30% dimethylsulfoxide (DMSO), 70% NMP prior to activation.

Cleavage of the resin-bound peptide was accomplished by treating the peptide resin using a mixture of trifluoroacetic acid, phenol, thioanisole, ethanedithiol, and water (82.5:5:5:2.5:5) (reagent K). Following cleavage, the peptide-containing cleavage mixture was filtered off and the volume reduced in a rotary evaporator. Methyl-*t*-butylether was then added to precipitate the peptide. The precipitated crude peptide was collected by filtration, dried under vacuum, and purified by reversed phase HPLC. Fractions containing the desired product were identified by electrospray mass spectrometry, lyophilized, and stored desiccated at 4°C.

### Example 2

**Serological recognition of the peptide by HIV-1 group O sera. Peptide** 147 was evaluated for its ability to be recognized by anti-HIV-1 group O antibodies present in human serum from infected individuals. All group O samples were confirmed to be positive for HIV-1 group O by reverse transcriptase-polymerase chain reaction (RT-PCR) amplification of viral gp41 sequences using O-specific primers. The identity of the PCR product was further confirmed by DNA sequencing.

A preferred method for demonstrating antibody binding is the enzyme-linked immunosorbent assay (ELISA). This technique involves binding of the antigen to a solid phase, bringing the fluid to be tested into contact with the antigen-coated solid phase, washing away any unbound material, and then detecting bound antibodies with an enzyme-labeled second antibody. The presence of the enzyme is detected using a chromogenic or luminogenic substrate.

To evaluate biotinylated peptide 147, wells of a microtiter plate were coated with streptavidin at a concentration of 1 µg/ml in 50 mM carbonate buffer, pH 9.6 (200 µl/well). Excess unbound streptavidin was removed by washing several times with phosphate buffered saline (PBS). Biotinylated peptide 147 was dissolved in a minimal volume of pure DMSO and then diluted into PBS to a concentration of 0.5 µg/ml. Two hundred microliters of the peptide solution were added to each well and the peptide allowed to bind to the streptavidin. After removing unbound peptide by washing with PBS, serum samples obtained from HIV-1 group O-infected patients were added to the wells of the plate. After a suitable incubation period, unbound material was removed by washing with PBS. A dilution of an anti-IgG:horse radish peroxidase (HRP) conjugate was added to each well and incubated for 30 minutes at 37°C. Unbound material was removed by extensive washing. Substrate solution containing O-phenylenediamine (OPD) and H₂O₂ was added to each well and the plate was further incubated, protected from light. After a 30 minute incubation period at room temperature, the reaction was terminated by the addition of 50 µl of 4N H₂SO₄ and the absorbance of each well was read at 492 nm.

Peptide 147 was first tested by using undiluted (10 µl) serum samples. The results are shown in Table 2. The sample measurements are in optical density (OD).

**Table 2.**

| Recognition of peptide 147 using undiluted specimens. | |
|---|---|
| undiluted samples | OD₄₉₂ |
| negative control | 0.056 |
| ODS001 | 2.500 |
| ODS002 | 2.500 |
| ODS003 | 2.500 |
| ODS004 | 2.500 |
| ODS006 | 2.500 |
| ODS007a | 2.500 |
| ODS007b | 2.500 |
| ODS009 | 2.500 |
| type B sample | 1.926 |

Samples ODS007a and b are consecutive bleeds from the same individual. The negative control sample is from an uninfected blood donor. These results show that antibodies produced in response to an HIV-1 group O infection are capable of recognizing peptide 147.

A panel of serially diluted group O sera was tested with peptide 147 in order to evaluate the sensitivity of an ELISA using this antigen. Streptavidin-coated plates were prepared and loaded with peptide 147 as before, and tested using the dilutional panels. In addition, in order to compare the performance of peptide 147 with that of a genuine group O sequence, a second peptide was synthesized (peptide 146) corresponding to the sequence of the DUR variant.

These peptides cover precisely the same region of gp41, and were tested in parallel. These results are shown in Table 3:

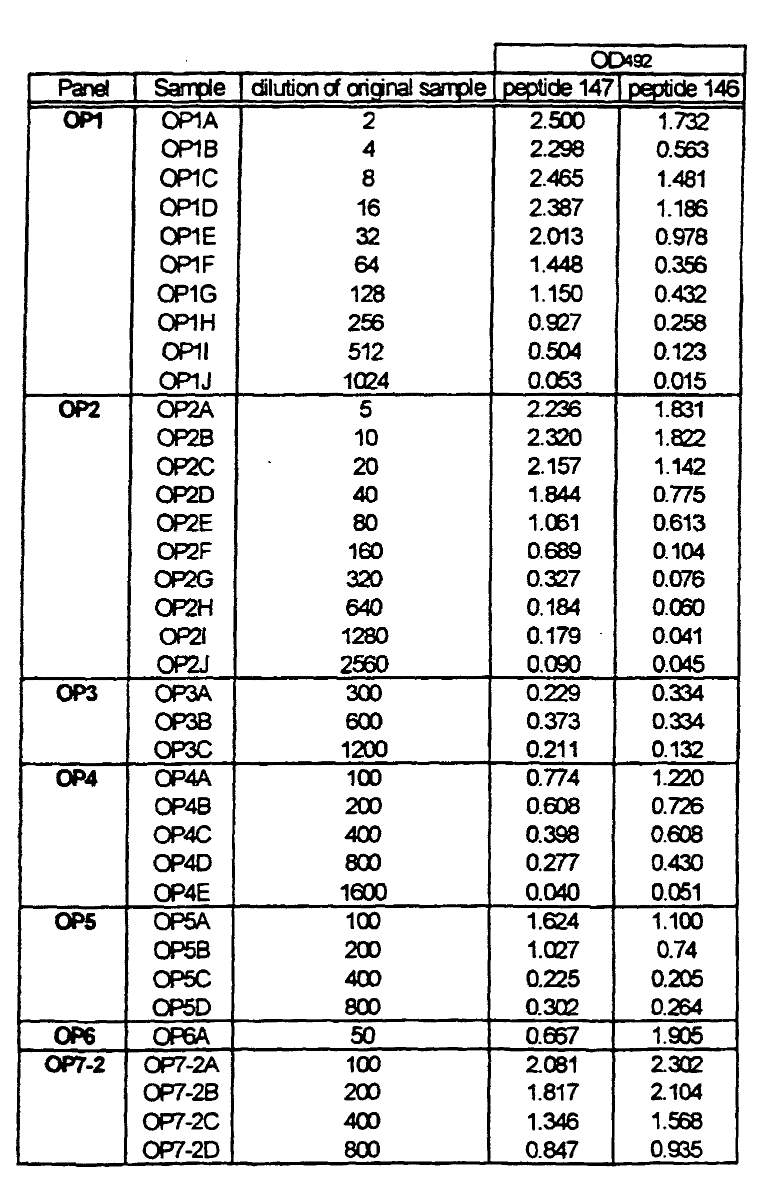

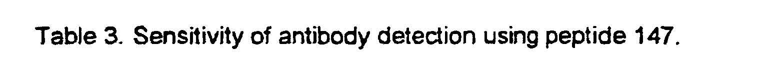

These results demonstrate that even with highly diluted samples, peptide 147 is a suitable antigen capable of binding antibodies against the group O gp41 immunodominant region. With a number of the panels, the performance of peptide 147 equaled or significantly exceeded that of a genuine group O sequence.

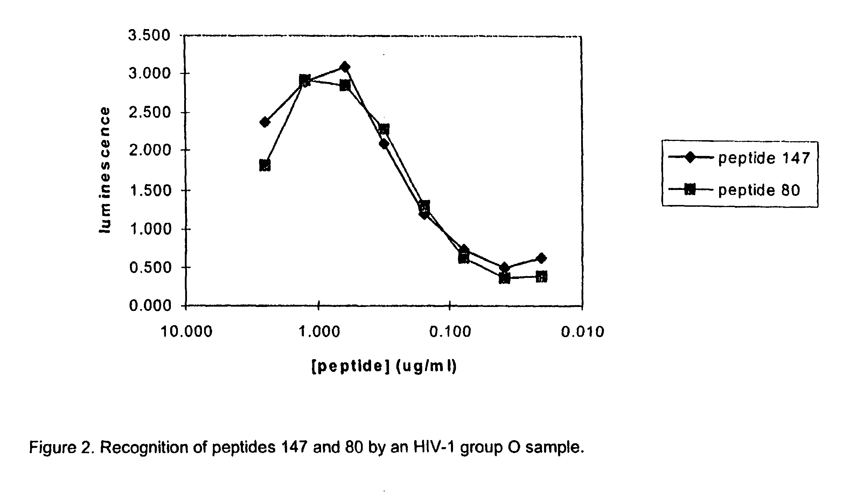

To compare the performance of peptide 147 to another genuine group O sequence, the peptide corresponding to the ANT70 sequence was synthesized (peptide 80). Peptides 147 and 80 were dissolved at the same concentration, serially diluted, and bound to streptavidin-coated wells. A 1:60 dilution of serum sample ODS007b (see Table 3) was added to all wells and incubated. After washing, an anti-human IgG:HRP conjugate was added, incubated, and the wells again thoroughly washed. In this case, a chemiluminescent substrate was used, consisting of luminol and a peracid salt. The emitted light was quantified using a luminometer. The results are tabulated in Table 4 and shown graphically in Figure 2.

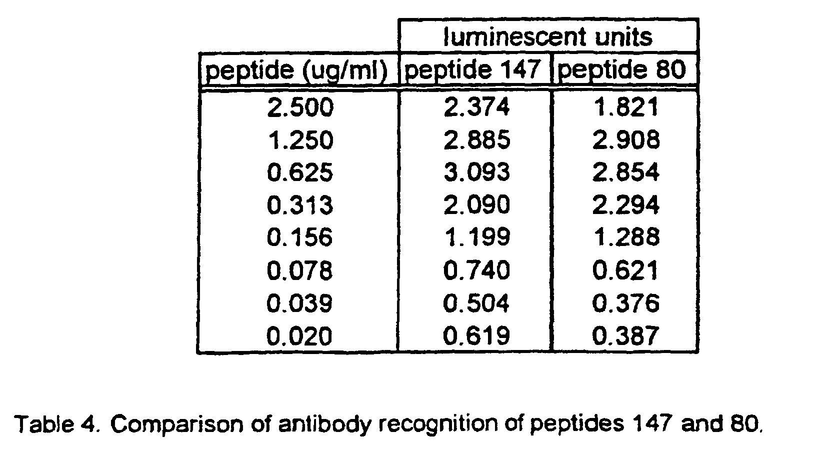

These results demonstrate that the amino acid sequence of peptide 147 is comparable to that of a naturally occurring isolate for the detection of antibodies to HIV-1 group O.

### Example 3

Antibody recognition of variants of peptide 147:

The following two peptides were synthesized:

Peptide 147-4:
EQQRLNSWGCKGRIICYTSARWH

Peptide 147-5:
GRETLMQDQQRLNSWGCKGRIICYTSARWH

In peptide 147-5, the N-terminal glycine was added to function as a spacer group, while the arginine in position 2 was added to enhance the solubility of the peptide in aqueous buffers. Both of these peptides were evaluated for their ability to be recognized by HIV-1 group O serum samples in an antibody capture format. In general, in such an assay, a peptide is bound to a solid phase and bivalent antibody presents in a sample binds to the peptide linked thereto. Bound antibody is subsequently detected using a conjugate made of the peptide in question coupled to horseradish peroxidase. The peptide-HRP conjugate binds to antibody which in turn is bound to the solid phase linked peptide. Methods suitable for preparing such conjugates may be found readily in the scientific literature and are well known to those versed in the art.

Microwell plates were pre-coated with either peptide 147-4 or 147-5. A dilution of a serum sample was then incubated for 1 hour at 37°C, after which the plate was washed extensively. A 1:100 dilution of either peptide 147-4 or 147-5 conjugated to HRP was then added and allowed to incubate for an additional hour at 37°C. Following this incubation, the plates were again thoroughly washed. The presence of bound conjugate was detected by addition of substrate for HRP (O-phenylenediamine) and H₂O₂. Antibody recognition of peptides 147-4 and 147-5 is shown in Table 5 below.

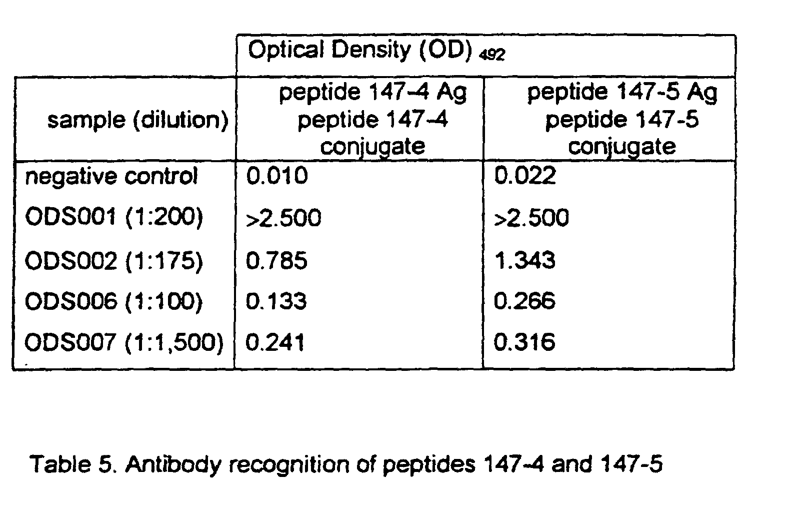

The peptides were evaluated for their ability to be recognized by anti-HIV-1 group O antibodies present in human serum from infected individuals. All group O samples were confirmed to be positive for HIV-1 group O by reverse transcriptase-polymerase chain reaction (RT-PCR) amplification of viral gp41 sequences using O-specific primers. The identity of the PCR product was further confirmed by DNA sequencing.

The results presented in the table demonstrate that the noted sequence variations of peptide 147 are suitable for detecting the presence of antibodies produced in response to an HIV-1 group O infection, even when the serum samples are highly diluted. The results also indicate that the use of the longer peptide results in a somewhat higher signal than the shorter version.

It is possible to introduced variations to the basic amino acid sequence of peptide 147 and retain the ability to detect antibodies produced in response to infection by HIV-1 group O.

### Example 4

**Design of a recombinant antigen for antibody detection.** In many cases, larger proteins often function better than short peptides for the detection of antibodies because they are able to present discontinuous epitopes created by the juxtaposition of distant regions of the polypeptide chain in the native protein. Other advantages to using recombinant proteins include the ability to incorporate into the protein specific sequences unrelated to the actual antigen in order to facilitate purification of the protein, binding to the solid phase, or to provide the protein with other desirable characteristics.

The sequence of the group M HIV-1 type B isolate MN was used as a point of departure in designing a novel antigen with desirable characteristics for the detection of anti-group O antibodies. The *env* gene product is a polypeptide 855 amino acids in length. Cleavage of the gp160 precursor occurs between amino acids 513 and 514 to give rise to the envelope proteins gp120 and gp41 found in mature virions. Of particular interest for antibody detection is the ectodomain of gp41, shown in Figure 3.

The design of a new recombinant protein should take into account any available structural information to avoid creating a protein that is intrinsically hindered in its ability to re-form its native structure in solution. The structures 1AIK and 1 ENV were obtained from the Brookhaven Protein Database and viewed using the programs Rasmol and SwissModel. These crystallographic structures show the presence of two interacting helices which form a coiled coil. The sequences connecting these two helices in the 1ENV structure (and which encompass the immunodominant region) are absent, suggesting that the interconnecting region comprising the immunogenic region at the top of a loop is too mobile to generate reflections. The helices for which there is crystallographic data are indicated in Figure 3. The N- and C-terminal limits to each helix are the limits for which crystallographic data is available; they are not necessarily the true boundaries of these helices in the native gp41 protein.

The potential importance of sequences contained within the "descending" helix shown in Figure 3 has been demonstrated for group O sera and are described in WO 95/32293. To determine which amino acids in the MN sequence should not be mutated, the available 3-dimensional structures were examined to identify those amino acids likely to be involved in stabilizing the interactions between the two helices. The following dose contacts were identified:
G548 → N657
Q551 → Q653
Q552 → Q654
A562 → I643
H565 → Y639
L569 → I636
W572 → W632
W572 → W629

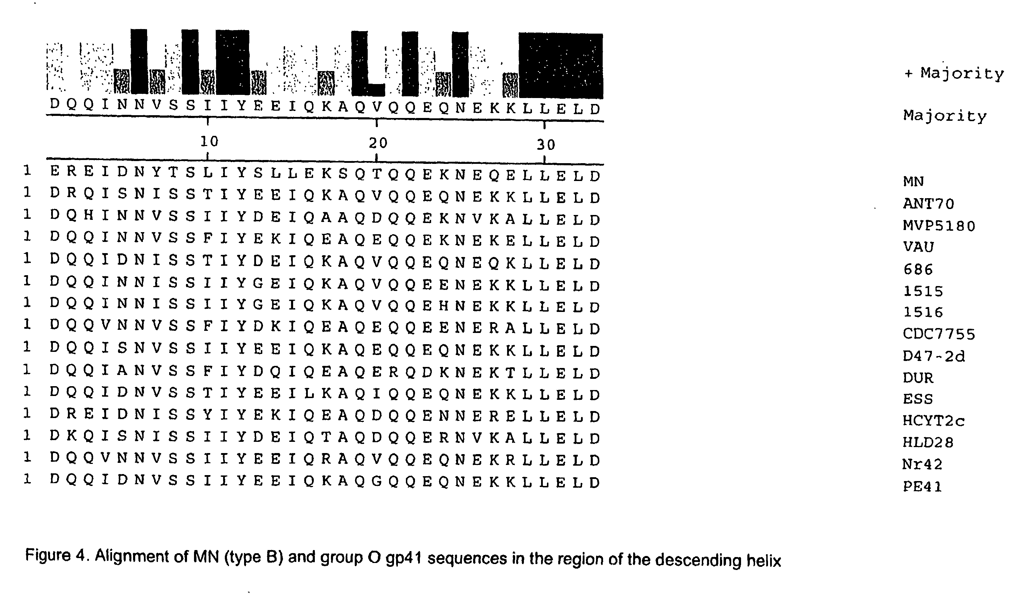

An examination of available sequences in the HIV Sequence Data Base maintained by the Los Alamos National Laboratory reveals that many of these amino acids are highly conserved. Mutations, when present, often have similar characteristics, such as a hydrophobic side-chain or a side-chain with the ability to participate in hydrogen bonding. The sequence of the MN isolate in the region of the descending helix (amino acids 633 - 665) was aligned and compared to that of all group O strains for which sequence information is available. This alignment is shown in Figure 4.

Figure 4 also shows which amino acids in this region are conserved, even between the type B sequence and the group O sequences. This sequence comparison suggests very strongly that there is a high degree of structural homology between group M and group O strains.

To arrive at a sequence representative of group O, only the group O sequences were considered. The sequence chosen after evaluation of the amino acids found in each position is as follows:

DQQVNNVSSIIYDKILEAQDQQEENVRELLELD

This sequence has 57.6% identity to the corresponding ANT70 sequence.

Additionally, the peptide 147 group O-like immunodominant region was extended in the N-terminal direction. This larger immunodominant region has the following sequence:

RARLQALETLMQNQQRLNSWGCKGRIICYTSARWH

With the N-terminal extension, this new sequence has 68.6% identity with the original ANT70 strain.

It would be understood by one skilled in the art that a diagnostic test could be developed using either the peptides or derivatives thereof as described herein to detect antibodies found in an infected patient, or using antibodies raised against the aforementioned peptides to detect antigen found in an infected patient.

### Example 5

**Construction of a "mosaic" group M/group O recombinant antigen.** Having taken structural features of gp41 into account and knowing that the sequence contained within peptide 147 is suitable for the detection of anti-group O antibodies, the construction of a mosaic recombinant was undertaken. The objective was to use the MN gp41 sequence as a point of departure and substitute the two group O regions described above, thereby creating a group O antigen using the type B MN sequence as a carrier for the immunologically important domains.

The strategy for constructing such a recombinant is shown in Figures 5a - c. The stepwise reconstruction and replacement of the immunodominant region using overlapping synthetic oligonucleotides is illustrated, along with all important restriction enzyme cleavage sites.

The sequences of the primers used are given in Figure 5d.

Two constructs were made as dihydrofolate reductase (DHFR) fusion proteins. Neither of the constructs represents a full-length gp41 molecule, however, the longer of the two (DHFR-hENV-MH) contains essentially the entire ectodomain of gp41 except for the N-terminal fusion peptide. The construct DHFR-hES-MH is a truncated version of the longer fusion protein. The amino acid sequences of these fusion proteins are shown in Figure 6, a. and b.

A C-terminal sequence derived from the *myc* gene was added to facilitate analysis, and a his₆ tail was added to facilitate purification. All non-HIV sequences are shown in boxes. The group O-like sequences are underlined. All other sequences in the HIV portion of this recombinant are group are group M-derived.

The expression of both proteins was attempted in *Escherichia coli.* The greatest level of expression was observed for the truncated construct DHFR-hES-MH and the expressed recombinant protein was found in inclusion bodies. The cells were lysed, and the inclusion bodies harvested by well known methods. The protein was subsequently solubilized by treatment with sodium dodecyl sulfate (SDS) and dithiothreitol, and analyzed by polyacrylamide gel electrophoresis in the presence of SDS. A band was observed having the expected molecular size and was estimated to constitute about 90% of the total protein in the crude antigen preparation.

### Example 6

**Performance of group M/group O "mosaic" constructs for group O antibody detection.** The protein expressed as described was coated at a concentration of 0.5 µg/ml into the wells of microtiter plates for evaluation. Following coating of the protein, any remaining binding sites on the plastic were block with bovine serum albumin. Dilutions of group O sera were then tested for their ability to recognize the recombinant antigen. Bound antibodies were detected by incubation with HRP-labeled mouse anti-human IgG conjugate, and the bound conjugate was subsequently detected by addition of OPD and H₂O₂. The results are shown in Table 6.

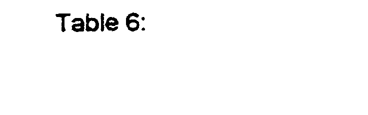

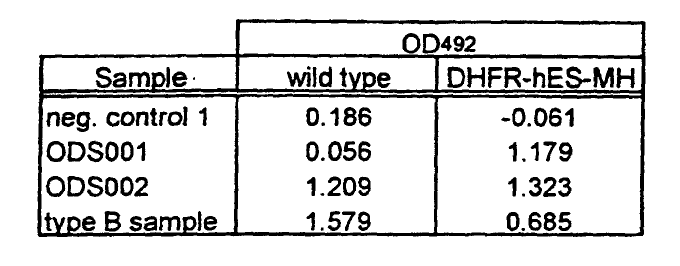

Because these antigens were derived by taking into consideration a reasonably large number of HIV-1 group O sequences, the new sequences are thought to better represent the spectrum of group O variants which are likely to occur in nature and may therefore have a broader specificity for the detection of anti-group O antibodies. Experimental results show that these antigens function as well, and in some cases better, than naturally occurring viral sequences for the detection of antibodies generated in response to HIV-1 group O infections.

The use of "mosaic" recombinants offer advantages in that they display the desired antigenic determinants but can be more easily bound by direct coating to a solid phase than peptides for use in an immunoassay. Furthermore, the recombinants, because of their increased length and more stabilized structure relative to peptides, present the determinants more accurately.

All cited materials herein are hereby incorporated by reference. Accordingly, it should be noted that the present invention includes all modifications falling within the scope of the following claims.

### Literature cited

1. Barre-Sinoussi, F., et al., **Science** (1983) 220:868-871.
2. Clavel, F., et al., **Science** (1986) 233:343-346.
3. Goodenow, M., et al., **J. Acquir. Immun. Defic. Syndr.** (1989) 2:344-352.
4. Leitner, T., et al., **Human Retroviruses and AIDS Compendium 1997** (1998), part III, pp. 19-24.
5. DeLeys, R., et al., **J. Virol.** (1990) 64:1207-1216.
6. Vanden Haesevelde, M., et al., **J. Virol.** (1994) 68:1586-1596.
7. Gurtler, L.G., et al., **J. Virol.** (1994) 68:1581-1585.
8. Charneau, P., et al., **Virology** (1994) 205:247-253.
9. Loussert-Ajaka, I., et al., **J. Virol.** (1995) 69:5640-5649.
10. Mauclère, P., et al., **AIDS** (1997) 11:445-453.
11. Peeters, M., et al., **AIDS** (1997) 11:493-498.
12. Loussert-Ajaka, I., et al., **Lancet** (1994) 343:1393-1394.
13. Britvan, L., et al., **MMWR** (1996) 45:561-565.
14. Heyndrickx, L., et al., **Lancet** (1996) 347:902-903.
15. Takehisa, J., et al., **J. Acquir. Immun. Defic. Syndr.** (1997) 14:81-82.
16. Hermanson, G.T.; **Bioconjugate Techniques,** Academic Press, San Diego, U.S.A., 1996.
17. Delaporte, E., et al., **AIDS** (1996) 10:903-910.
18. Brennan, C., et al., **AIDS Res. Human. Retrovir.** (1997) 13:901-904.
19. Hackett, J., et al., **Fourth Conf. on Retroviruses and Opportun. Infect.,** Washington, D.C. (1997), abstr. 160.

## Claims

1. A peptide comprising an amino acid sequence selected from the group consisting of: 
NQQRLNSWGCKGRIICYTSARWH,
EQQRLNSWGCKGRIICYTSARWH,
GRETLMQDQQRLNSWGCKGRIICYTSARWH
XQQRLNSWGCKGRIICYTSARWH,
ETLMQXQQRLNSWGCKGRIICYTSARWH,
RARLQALETLMQNQQRLNSWGCKGRIICYTSARWH,
 werein X denotes any natural amino acid other tran L-asparagine, or a non-natural amino acid.

2. The peptide of claim 1 wherein said peptide is antigenic.

3. The peptide of claim 1 or claim 2 wherein said peptide binds anti-HIV group O antibodies.

4. The peptide of any one of claims 1 to 3 wherein said peptide is made by recombinant or synthetic chemistry methods.

5. An antibody raised against the peptide of any one of claims 1 to 4.

6. A nucleic acid sequence that encodes the peptide of any one of claims 1 to 4.

7. A vector for expression containing the nucleic acid sequence of claim 6.

8. A host cell containing the expression vector of claim 8.

9. A process for expression of a peptide in a recombinant host cell, comprising: (a) transferring the expression vector of claim 7 into suitable host cells, and (b) culturing the host cells of step (a) under a condition which allows expression of the peptide from the expression vector.

10. A test kit comprising one or more peptides selected from the group consisting of: NQQRLNSWGCKGRIICYTSARWH, EQQRLNSWGCKGRIICYTSARWH, GRETLMQDQQRLNSWGCKGRIICYTSARWH XQQRLNSWGCKGRIICYTSARWH, ETLMQXQQRLNSWGCKGRIICYTSARWH, RARLQALETLMQNQQRLNSWGCKGRIICYTSARWH, and antibodies that bind to said peptides, wherein X denotes any natural amino acid other than L-asparagine, or a non-natural amino acid.

11. An in vitro diagnostic assay method comprising contacting a sample with one or more peptides selected from the group consisting of: 
NQQRLNSWGCKGRIICYTSARWH, EQQRLNSWGCKGRIICYTSARWH,
GRETLMQDQQRLNSWGCKGRIICYTSARWH
XQQRLNSWGCKGRIICYTSARWH,
ETLMQXQQRLNSWGCKGRIICYTSARWH,
RARLQALETLMQNQQRLNSWGCKGRIICYTSARWH,
 and determining binding between said peptide and an antibody, wherein X denotes any natural amino acid other than L-asparagine, or a non-natural amino acid.

12. An in vitro diagnostic assay method comprising contacting a sample with one or more antibodies raised against a peptide of claim 1 and determining binding between said antibodies and an antigen.

13. A mosaic comprising a recombinant group M gp 41 protein wherein a group M immunodominant region has been replaced by one or more O-like immunodominant sequences, wherein the O-like immunodominant sequence is selected from the group consisting of: 
NQQRLNSWGCKGRIICYTSARWH, EQQRLNSWGCKGRIICYTSARWH,
GRETLMQDQQRLNSWGCKGRIICYTSARWH
XQQRLNSWGCKGRIICYTSARWH,
ETLMQXQQRLNSWGCKGRIICYTSARWH,
RARLQALETLMQNQQRLNSWGCKGRIICYTSARWH,
 wherein X denotes any natural amino acid other than L-asparagine, or a non-natural amino acid.

14. A mosaic comprising a recombinant group M gp 41 protein wherein a group M immunodominant region has been replaced by one or more O-like immunodominant sequences wherein said mosaic is selected from the group consisting of: 
ARLLLSGIVQQQNNLLRAIEAQQHMLQLTAWGIKQLRARLQALETLMQN
QQRLNSWGCKGRIICYTSARWHASWSNKSLEDIWDNMTWMQWDQQ
VNNVSSIIYDKILEAQDQQEENVRELLELDKWASLWNWFDITNWLWYIKI
FIMIVGGLVGLRIVFAVLSIVNRVRQGYSPLSLQTRPPVPRGPDRPEGIE
EEGGERDRDTSGRLVHGFLAIIWVDL
 and

ARLLLSGIVQQQNNLLRAIEAQQHMLQLTAWGIKQLRARLQALETLMQN
QQRLNSWGCKGRIICYTSARWHASWSNKSLEDIWDNMTWMQWDQQ
VNNVSSIIYDKILEAQDQQEENVRELLELDKWASLWNWFDITNWLWYIKI
FIMIV.

## Patentansprüche

1. Ein Peptid, umfassend eine Aminosäuresequenz ausgewählt aus der Gruppe bestehend aus: 
NQQRLNSWGCKGRIICYTSARWH,
EQQRLNSWGCKGRIICYTSARWH,
GRETLMQDQQRLNSWGCKGRIICYTSARWH
XQQRLNSWGCKGRIICYTSARWH,
ETLMQXQQRLNSWGCKGRIICYTSARWH,
RARLQALETLMQNQQRLNSWGCKGRIICYTSARWH,
 worin X jede natürliche Aminosäure anders als L-Asparagin oder eine nicht-natürliche Aminosäure bezeichnet.

2. Peptid nach Anspruch 1, wobei das Peptid antigen ist.

3. Peptid nach Anspruch 1 oder Anspruch 2, wobei das Peptid Anti-HIV-Gruppe O Antikörper bindet.

4. Peptid nach einem der Ansprüche 1 bis 3, wobei das Peptid durch rekombinante oder synthetische chemische Verfahren hergestellt wird.

5. Antikörper, erzeugt gegen das Peptid nach einem der Ansprüche 1 bis 4.

6. Nukleinsäuresequenz, die für ein Peptid nach einem der Ansprüche 1 bis 4 kodiert.

7. Vektor zur Expression, enthaltend die Nukleinsäuresequenz nach Anspruch 6.

8. Wirtszelle, enthaltend den Expressionsvektor nach Anspruch 8.

9. Verfahren zur Expression eines Peptids in einer rekombinanten Wirtszelle, umfassend: (a) Transferieren des Expressionsvektors nach Anspruch 7 in geeignete Wirtszellen und (b) Kultivieren der Wirtszellen von Schritt (a) unter einer Bedingung, die die Expressions des Peptids von dem Expressionsvektor ermöglicht.

10. Testkit, umfassend ein oder mehrere Peptide ausgewählt aus der Gruppe bestehend aus: 
NQQRLNSWGCKGRIICYTSARWH,
EQQRLNSWGCKGRIICYTSARWH,
GRETLMQDQQRLNSWGCKGRIICYTSARWH
XQQRLNSWGCKGRIICYTSARWH,
ETLMQXQQRLNSWGCKGRIICYTSARWH,
RARLQALETLMQNQQRLNSWGCKGRIICYTSARWH,
 und Antikörpern, die an die Peptide binden, wobei X jede natürliche Aminosäure anders als L-Asparagin oder eine nicht-natürliche Aminosäure bezeichnet.

11. In vitro diagnostisches Testverfahren, umfassend in-Kontakt-Bringen einer Probe mit einem oder mehreren Peptiden, ausgewählt aus der Gruppe bestehend aus: 
NQQRLNSWGCKGRIICYTSARWH, EQQRLNSWGCKGRIICYTSARWH,
GRETLMQDQQRLNSWGCKGRIICYTSARWH
XQQRLNSWGCKGRIICYTSARWH,
ETLMQXQQRLNSWGCKGRIICYTSARWH,
RARLQALETLMQNQQRLNSWGCKGRIICYTSARWH,
 und Bestimmen der Bindung zwischen dem Peptid und einem Antiköper, wobei X jede natürliche Aminosäure anders als L-Asparagin oder eine nicht-natürliche Aminosäure bezeichnet.

12. In vitro diagnostisches Testverfahren, umfassend In-Kontakt-Bringen einer Probe mit einem oder mehreren Antikörpern, erzeugt gegen ein Peptid nach Anspruch 1 und Bestimmen der Bindung zwischen den Antikörpern und einem Antigen.

13. Mosaik, umfassend ein rekombinantes Gruppe M gp 41 Protein, wobei eine Gruppe M immundominante Region durch eine oder mehrere O-ähnliche immundominante Sequenzen ersetzt wurde, wobei die O-ähnliche immundominante Sequenz ausgewählt ist aus der Gruppe bestehend aus: 
NQQRLNSWGCKGRIICYTSARWH, EQQRLNSWGCKGRIICYTSARWH,
GRETLMQDQQRLNSWGCKGRIICYTSARWH
XQQRLNSWGCKGRIICYTSARWH,
ETLMQXQQRLNSWGCKGRIICYTSARWH,
RARLQALETLMQNQQRLNSWGCKGRIICYTSARWH,
 worin X jede natürliche Aminosäure anders als L-Asparagin oder eine nicht-natürliche Aminosäure bezeichnet.

14. Mosaik, umfassend ein rekombinantes Gruppe M gp 41 Protein, wobei eine Gruppe M immundominante Region durch eine oder mehrere O-ähnliche immundominante Sequenzen ersetzt wurde, wobei das Mosaik ausgewählt ist aus der Gruppe bestehend aus: 
ARLLLSGIVQQQNNLLRAIEAQQHMLQLTAWGIKQLRARLQALETLMQN
QQRLNSWGCKGRIICYTSARWHASWSNKSLEDIWDNMTWMQWDQQ
VNNVSSIIYDKILEAQDQQEENVRELLELDKWASLWNWFDITNWLWYIKI
FIMIVGGLVGLRIVFAVLSIVNRVRQGYSPLSLQTRPPVPRGPDRPEGIE
EEGGERDRDTSGRLVHGFLAIIWVDL
 und

ARLLLSGIVQQQNNLLRAIEAQQHMLQLTAWGIKQLRARLQALETLMQN
QQRLNSWGCKGRIICYTSARWHASWSNKSLEDIWDNMTWMQWDQQ
VNNVSSIIYDKILEAQDQQEENVRELLELDKWASLWNWFDITNWLWYIKI
FIMIV.

## Revendications

1. Peptide comprenant une séquence d'acides aminés choisie dans le groupe constitué de : 
NQQRLNSWGCKGRIICYTSARWH,
EQQRLNSWGCKGRIICYTSARWH,
GRETLMQDQQRLNSWGCKGRIICYTSARWH,
XQQRLNSWGCKGRIICYTSARWH,
ETLMQXQQRLNSWGCKGRIICYTSARWH,
RARLQALETLMQNQQRLNSWGCKGRIICYTSARWH,
 où X indique un acide aminé naturel quelconque autre que la L-asparagine ou un acide aminé non naturel.

2. Peptide selon la revendication 1, où ledit peptide est antigénique.

3. Peptide selon la revendication 1 ou la revendication 2, où ledit peptide se lie aux anticorps anti-VIH du groupe O.

4. Peptide selon l'une quelconque des revendications 1 à 3, où ledit peptide est fabriqué par des procédés de recombinaison ou de synthèse chimique.

5. Anticorps dirigé contre le peptide selon l'une quelconque des revendications 1 à 4.

6. Séquence d'acide nucléique qui code pour le peptide selon l'une quelconque des revendications 1 à 4.

7. Vecteur d'expression contenant la séquence d'acide nucléique selon la revendication 6.

8. Cellule hôte contenant le vecteur d'expression selon la revendication 7.

9. Procédé d'expression d'un peptide dans une cellule hôte recombinante, comprenant : (a) le transfert du vecteur d'expression selon la revendication 7 dans des cellules hôtes CDS-182 appropriées et (b) la culture des cellules hôtes de l'étape (a) dans des conditions qui permettent l'expression du peptide à partir du vecteur d'expression.

10. Kit de test comprenant un ou plusieurs peptides choisis dans le groupe constitué de : 
NQQRLNSWGCKGRIICYTSARWH,
EQQRLNSWGCKGRIICYTSARWH,
GRETLMQDQQRLNSWGCKGRIICYTSARWH,
XQQRLNSWGCKGRIICYTSARWH,
ETLMQXQQRLNSWGCKGRIICYTSARWH,
RARLQALETLMQNQQRLNSWGCKGRIICYTSARWH,
 et d'anticorps qui se lient aux dits peptides, où X indique un acide aminé naturel quelconque autre que la L-asparagine ou un acide aminé non naturel.

11. Procédé de test de diagnostic *in vitro* comprenant la mise en contact d'un échantillon avec un ou plusieurs peptides choisis dans le groupe constitué de : 
NQQRLNSWGCKGRIICYTSARWH,
EQQRLNSWGCKGRIICYTSARWH,
GRETLMQDQQRLNSWGCKGRIICYTSARWH,
XQQRLNSWGCKGRIICYTSARWH,
ETLMQXQQRLNSWGCKGRIICYTSARWH,
RARLQALETLMQNQQRLNSWGCKGRIICYTSARWH,
 et la détermination de la liaison entre ledit peptide et un anticorps, où X indique un acide aminé naturel quelconque autre que la L-asparagine ou un acide aminé non naturel.

12. Procédé de test de diagnostic in vitro comprenant la mise en contact d'un échantillon avec un ou plusieurs anticorps dirigés contre un peptide selon la revendication 1 et la détermination de la liaison entre lesdits anticorps et un antigène.

13. Mosaïque comprenant une protéine gp 41 du groupe M recombinante, où une région immunodominante du groupe M a été remplacée par une ou plusieurs séquences immunodominantes semblables au groupe O, où la séquence immunodominante semblable au groupe O est choisie dans le groupe constitué de : 
NQQRLNSWGCKGRIICYTSARWH,
EQQRLNSWGCKGRIICYTSARWH,
GRETLMQDQQRLNSWGCKGRIICYTSARWH,
XQQRLNSWGCKGRIICYTSARWH,
ETLMQXQQRLNSWGCKGRIICYTSARWH,
RARLQALETLMQNQQRLNSWGCKGRIICYTSARWH,
 où X indique un acide aminé naturel quelconque autre que la L-asparagine ou un acide aminé non naturel.

14. Mosaïque comprenant une protéine gp 41 du groupe M recombinante, où une région immunodominante du groupe M a été remplacée par une ou plusieurs séquences immunodominantes semblables au groupe O, où ladite mosaïque est choisie dans le groupe constitué de : 
ARLLLSGIVQQQNNLLRAIEAQQHMLQLTAWGIKQLRARLQALETLMQN
QQRLNSWGCKGRIICYTSARWHASWSNKSLEDIWDNMTWMQWDQQ
VNNVSSIIYDKILEAQDQQEENVRELLELDKWASLWNWFDITNWLWYIKI
FIMIVGGLVGLRIVFAVLSIVNRVRQGYSPLSLQTRPPVPRGPDRPEGIE
EEGGERDRDTSGRLVHGFLAIIWVDL
 et
